# EUROPÄISCHE PATENTANMELDUNG

(11) **EP 1 008 584 A1**
(43) Veröffentlichungstag der Anmeldung: **14.06.2000**
(21) Anmeldenummer: 99102080.1
(22) Anmeldetag: 02.02.1999
(51) Int. Cl.: C07C 67/00, C07C 69/017, C07C 69/157, C07C 39/08, C07C 37/01

(54) **Verfahren zur Herstellung von Trimethylhydrochinondiestern und von Trimethylhydrochinon**

(30) Priorität: 12.02.1998 DE 19805690
(71) Anmelder: Degussa Aktiengesellschaft, 60311 Frankfurt (DE)
(72) Erfinder: Krill, Steffen, Dr., 67346 Speyer (DE); Weigel, Horst, 63517 Rodenbach (DE); Shi, Nongyuan, Dr., 63450 Hanau (DE); Hasselbach, Hans-Joachim, Dr., 63571 Gelnhausen (DE); Huthmacher, Klaus, Dr., 63584 Gelnhausen (DE); Hübner, Frank, Dr., 64372 Ober-Ramstadt (DE)

(57) **Zusammenfassung**

Die Erfindung betrifft ein verbessertes Verfahren zur Herstellung von 2,3,5-Trimethylhydrochinon durch Umlagerung von 4-Oxo-Isophoron (Keto-Isophoron, 3,5,5-Trimethylcyclohex-2-en-1,4-dion) zu einem Trimethylhydrochinondiester und dessen anschließende Verseifung.

Trimethylhydrochinon wiederum ist ein wichtiges Ausgangsprodukt für die Herstellung von Vitamin E.

## Beschreibung

Die Erfindung betrifft ein verbessertes Verfahren zur Herstellung von 2,3,5-Trimethylhydrochinon und 2,3,5-Trimethylhydrochinondiestern durch Umsetzung von 4-Oxo-Isophoron (Keto-Isophoron, 3,5,5-Trimethylcyclohex-2-en-1,4-dion) mit einem Acylierungsmittel in Gegenwart katalytischer Mengen einer Supersäure zu einem Trimethylhydrochinondiester der gegebenenfalls anschließend zum Trimethylhydrochinon verseift wird.

Trimethylhydrochinon wiederum ist ein wichtiges Ausgangsprodukt für die Herstellung von Vitamin E.

### Stand der Technik

Es ist bereits bekannt (DE 26 46 172 C2), Keto-Isophoron in der Gasphase mit einem Zeolithen in das Trimethylhydrochinon umzulagern. Die Ausbeuten bei dieser Reaktion sind allerdings nur gering (50 % bei 30 % Umsatz) und damit für ein ökonomisches Verfahren unbefriedigend. In einem weiteren Verfahren (Y.A. Joe, Y.M. Goo, Y.Y. Lee *Bull. Korean. Chem. Soc.* 1991, 12, 253) wird die Umlagerung in 5 %-iger Lösung in Acetanhydrid durch Zugabe von fünf Äquivalenten konzentrierter Schwefelsäure durchgeführt. Trimethylhydrochinonester werden dabei mit lediglich 31 % Ausbeute erhalten, so daß auch dieses Verfahren nicht wirtschaftlich ist.
Nach einer dritten Methode (DE-OS 2 149 159) kann Keto-Isophoron in Gegenwart einer Protonensäure in Acetanhydrid zu Trimethylhydrochinondiacetat umgesetzt werden, welches anschließend zu 2,3,5- Trimethylhydrochinon verseift wird. Nachteilig an diesem Verfahren ist die Verwendung
- großer Mengen an Acetanhydrid (5-10 mol/mol Ketoisophoron),
- großer Mengen der Katalysatorsäure (bis 150 mol%)
- sowie die mit maximal 66 % recht mäßige Ausbeute.

Es wurde nun ein Verfahren zur Herstellung von Trimethylhydrochinon (TMHQ)durch Umsetzung von Keto-Isophoron mit einem Acylierungsmittel in Gegenwart katalytischer Mengen einer Protonensäure und anschließende Verseifung des zunächst gebildeten Trimethylhydrochinonesters gefunden, das dadurch gekennzeichnet ist, daß man eine Protonensäure mit einer Hammett-Konstante Hₒ < -11,9 einsetzt.
Säuren dieses Typs werden allgemein auch als 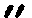 supersaure Sauren" oder 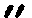 Supersäuren" bezeichnet.
Eine Beschreibung findet sich bei
*Olah et al., Science Nr. 4414, Vol 206, 13 ff (1979) und Gillespie et al., J. Am. Chem. Soc., Vol 93, 5083 ff (1971)*.
Explizit zu nennen und geeignet für die Verwendung im beanspruchten Verfahren sind Perchlorsäure, Fluorsulfonsäure und Perfluoralkansulfonsäuren der allgemeinen Formel

CₙF₂ₙ₊₁SO₃H (I),

in der n = 1-8 bedeutet

Geeignet sind auch Kombinationen oder Mischungen von Brönstedtsäuren mit Lewissäuren. Im Sinne der vorliegenden Erfindung sind dies Mischungen diverser Metallhalogenide, wie beispielsweise die Halogenide von Aluminium, Zink, Eisen, Antimon, Arsen, Niob, Tantal oder Wismut, mit verschiedenen Brönstedtsäuren.

Bevorzugt werden ebenso H₂SO₄-H₃BO₃ oder HB(HSO₄)₄-H₂SO₄, Mischungen von Halogensulfonsäure und Schwefelsäure. Systeme, die als aktive Katalysatorspezies H⁺AlCl₄⁻ oder H⁺BF₄⁻ freisetzen, Magic Acid (HSO₃F-SbF₅) oder Fluor-Antimon-Säure (HF-SbF₅) haben sich ebenfalls als sehr geeignet erwiesen.

Erfindungsgemäß liegen die Säuren gelöst vor.
Man setzt die Säuren in einer Menge von 0,1 bis 50 Gew.-%,insbesondere 0,5 bis 25 Gew.-%, bezogen auf das Endion, ein.
Bevorzugt verwendet man pro Mol Keto-Isophoron >2 bis 4 Mol, insbesondere 2,1 bis 3 mol eines der allgemein bekannten Acylierungsmittel.

Bei dem erfindungsgemäß verwendeten Acylierungsmittel handelt es sich vorzugsweise um ein Carbonsäureanhydrid, ein Carbonsäurechlorid, oder einen Enolester, insbesondere Diketen. Insbesondere wird ein Carbonsäureanhydrid der allgemeinen Formel 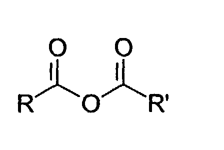 in der R und R' einen gegebenenfalls substituierten aliphatischen, alicyclischen oder aromatischen Rest mit 1 bis 8 Kohlenstoffatomen, der gegebenenfalls 1 bis 3 Halogenatome enthalten kann, bedeutet.

Ein besonders bevorzugt verwendetes Acylierungsmittel sind Essigsäureanhydrid und Essigsäurechlorid. Weitere geeignete Säureanhydride und Säurehalogenide sind Anhydride und Chloride von Propionsäure, Buttersäure, Isobuttersäure, Cyclohexancarbonsäure, Benzoesäure, Chloressigsäure, Trifluoressigsäure.

In einer bevorzugten Ausführungsform verseift man das entstandene TMHQ-Diacetat ohne Isolierung, gegebenenfalls nach dem Abdestillieren von nicht umgesetztem Acetanhydrid durch Zugabe von Wasser oder verdünnter Säure, insbesondere Schwefelsäure, und Erhitzen der Mischung zum Sieden. Das entstandene TMHQ wird anschließend abfiltriert.

Man kann aber auch das entstandene TMHQ-Diacetat nach der Zugabe von Wasser aus dem Reaktionsgemisch abtrennen und isolieren, in verdünnter Säure, insbesondere Schwefelsäure in Gegenwart eines Phasenvermittlers hydrolysieren und das entstandene TMHQ abtrennen, insbesondere durch Filtrieren.

Als Phasenvermittler bei der Verseifung auch des isolierten Trimethylhydrochinondiesters können alle organischen Lösungsmittel Verwendung finden, die eine gewisse Mischbarkeit mit Wasser aufweisen. Besonders vorteilhaft können Essigsäure, n-Butanol und n-Butylacetat oder Mischungen der genannten Lösungsmittel eingesetzt werden.

### Durchführung

Zur Herstellung von 2,3,5 - Trimethylhydrochinon nach dem erfindungsgemäßen Verfahren werden in einem Eintopfverfahren beispielsweise 0,2 Mol Keto-Isophoron zu einer Mischung von > 0,4-0,6 Mol Essigsäureanhydrid und 0,1- 50 Gew.-%,insbesondere 0,5 - 25 Gew.-%, bezogen auf Keto-Isophoron,einer der genannten sehr starken Säuren innerhalb von 1 bis 3 h bei 0 - 60 ° C zugetropft und anschließend 1 bis 7 h auf ca. 25 - 70 ° C erwärmt. Danach werden durch Zugabe einer ausreichenden Menge Wasser Reste des Essigsäureanhydrids hydrolysiert. Zu der entstandenen Suspension gibt man gegebenenfalls Schwefelsäure, bevorzugt ca. 30 %ige, und erhitzt 1 bis 5 h zum Sieden. Anschließend wird ein Teil des Lösungsmittels abdestilliert und durch Wasser ersetzt, die Suspension auf Raumtemperatur abgekühlt und das ausgefallene Trimethylhydrochinon abtrennt.

Man kann ebenso nach der ersten Zugabe von Wasser den ausgefallenen Trimethylhydrochinondiester abtrennen und separat verseifen. Dazu wird der Trimethylhydrochinondiester z. B. in einer ausreichenden Menge einer verdünnten Säure, bevorzugt 30 %iger Schwefelsäure, und einem Phasenvermittler, wie z. B. n-Butanol supendiert und anschließend 1 bis 7 h zum Sieden erhitzt.
Danach wird Destillat abgenommen und anschließend Wasser dem Sumpf zugesetzt. Das dann ausgefallene Trimethylhydrochinon wird abgetrennt und durch Nachwaschen gereinigt.

Die Erfindung wird im folgenden anhand von Ausführungsbeispielen weiter erläutert.

### Vorteile

Bei der erfindungsgemäßen Herstellung von 2,3,5-Trimethylhydrochinon ergeben sich erhebliche Vorteile gegenüber dem Stand der Technik:
- Die Ausbeuten sind nach dem erfindungsgemäßen Verfahren um bis zu 25 % höher als in der zitierten Literatur und liegen zwischen 85 und 95 %.
- Die benötigten Katalysatormengen liegen bei 0,1 bis 50 % gegenüber bis zu 150 % in der Literatur.
- Es werden lediglich >2 bis 4 Mol eines Carbonsäureanhydrids pro Mol Keto-Isophoron benötigt gegenüber 5 bis 10 Mol in der Literatur.

Die Verseifung des isolierten Trimethylhydrochinondiesters mit wäßriger Säure gelingt bevorzugt in einfacher Weise in Gegenwart eines Phasenvermittlers.

### Ausführungsbeispiele:

### Beispiel 1:

Zu 61 g (0,6 mol) Acetanhydrid gibt man 0,34 g (2,3 mmol) Trifluormethansulfonsäure und tropft unter Feuchtigkeitsausschluß 30,5 g (0,2 mol) Keto-Isophoron (98%) innerhalb 30 min zu. Durch Kühlen wird die Temperatur unter 50°C gehalten. Zum vollständigen Umsatz wird die Temperatur weitere 2 Stunden bei 45 bis 50°C gehalten. Danach wird auf 20°C abgekühlt, wobei sich Kristalle bilden. Zur vollständigen Kristallisation des Trimethylhydrochinon-diacetats werden 125 ml Wasser zugegeben. Der Feststoff wird abgesaugt und in einer Mischung aus 100ml 30%iger Schwefelsäure und 15 ml n-Butanol 4 Stunden zum Sieden erhitzt. Danach werden 80 ml einer Mischung aus Essigsäure, n-Butanol und Wasser abdestilliert, 100ml Wasser zugegeben und die Suspension wird auf 20°C abgekühlt. Das ausgefallene Trimethylhydrochinon wird abgesaugt, mit Wasser gewaschen und im Vakuum bei 55°C getrocknet.
- Ausbeute:: 27,3 g (89,7 % d. Th)
- Reinheit:: 98,5 % (HPLC)

### Beispiel 2:

133 mg (2,15 mmol) Borsäure und 860 mg (8,6 mmol) 98%ige Schwefelsäure werden in 30,6 g (0,3 mol)Acetanhydrid 30 Minuten gerührt und anschließend werden 15,5 g (0,1 mol) Keto-Isophoron (98%) zugetropft. Durch Kühlen wird die Temperatur unter 35°C gehalten. Anschließend wird die Temperatur 5 h bei 30°C gehalten, wobei 98,6 % der Ketoverbindung reagieren. Die Analyse (HPLC) ergibt einen Gehalt von 45,2 % Trimethylhydrochinon, was einer Selektivität von 91,5 % entspricht.

### Beispiel 3:

In 35,7 g (0,35 mol) Acetanhydrid werden 121 mg (1,95 mmol) Borsäure und 1,0 g (10 mmol) Schwefelsäure (98%) unter Feuchtigkeitsausschluß vorgelegt und 15,5 g (0,1 mol) Keto-Isophoron (98%) werden bei 30°C unter Rühren zugetropft. Es wird weitere 4,5 h bei 30°C gerührt. Danach gibt man 70 g 30%ige wäßrige Schwefelsäure zu und hydrolysiert durch Kochen unter Rückfluß. Nachdem 40 ml (Essigsäure, Wasser) abdestilliert sind, wird mit 50 ml Wasser verdünnt und auf 20°C gekühlt. Das auskristallisierte Trimethylhydrochinon wird abgesaugt, mit Wasser gewaschen und im Vakuum getrocknet.
- Ausbeute:: 13,9 g (91,4 % d. Th.) Trimethylhydrochinon
- Reinheit:: 95,8 % (HPLC)

### Beispiel 4:

31,4 g (0,4 mol) Acetylchlorid und 0,3 g Trifluermethansulfonsäure werden vorgelegt und 12,4 g (0,08 mol) Keto-Isophoron (98%) werden zugetropft, wobei HCl entweicht und die Temperatur von 25 auf 32°C steigt. Nach einer Stunde bei 50°C ist der Umsatz von Keto-Isophoron vollständig. Das überschüssige Acetylchlorid wird bei vermindertem Druck abdestilliert und der verbleibende Rückstand wird mit 50 ml Eiswasser verrührt. Nach Absaugen, Waschen mit Wasser und Trocknen bei 45°C im Vakuum erhält man 17,6 g Trimethylhydrochinon-diacetat (93,1 % d.Th.) mit einer Reinheit von 93,9% (HPLC).

### Beispiel 5:

Zu 38,8 g (0,38 mol) Acetanhydrid gibt man unter Feuchtigkeitsausschluß 0,26 ml (4,6 mmol) Fluersulfonsäure und tropft 23,3 g (0,15 mol Ketoisophoron (98%) zu. Die Mischung wird solange auf 50 - 60°C erwärmt, bis Keto-Isophoron zu über 99% reagiert hat. Danach wird die klare Lösung mit 130 g Eiswasser versetzt und mit 40%iger wäßriger Natronlauge auf PH 6 gebracht. Das kristallisierte Trimethylhydrochinon-diacetat wird bei 5°C abgesaugt und mit Wasser gewaschen. Nach dem Trocknen im Vakuum bei 50°C erhält man 34,7 g, was einer Ausbeute von 98,0% d. Th bedeutet. Die Reinheit beträgt 94,7 % (HPLC).

### Beispiel 6

20 ml konzentrierte Schwefelsäure werden mit 4,8 g Borsäure 20 Minuten gerührt, danach werden unter Kühlung 20 ml Oleum (65% SO3) zugetropft.

7,6 g dieser Borschwefelsäure werden zu 25,5 g (0,25 mol) Acetanhydrid gegeben und bei 40°C werden 15,5 g (0,1 mol) Keto-Isophoron (98%) zugetropft. Nach 2,5 Stunden bei dieser Temperatur ist der Umsatz vollständig. Die Selektivität der Bildung von Trimethylhydrochinon-diacetat beträgt 93,1% (GC). Nach der Hydrolyse, wie in Beispiel 3 beschrieben, werden 14,1 g Trimethylhydrochinon (92,8 % d. Th.) mit einer Reinheit von 96,0 % (HPLC) erhalten.

## Patentansprüche

1. Verfahren zur Herstellung von Trimethylhydrochinondiestern (2) 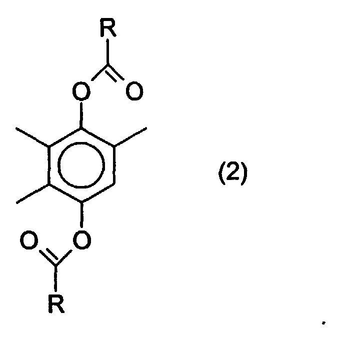 worin R einen gegebenenfalls substituierten aliphatischen, alicyclischen oder aromatischen Kohlenwasserstoffrest repräsentiert
und 2,3,5-Trimethylhydrochinon (3) 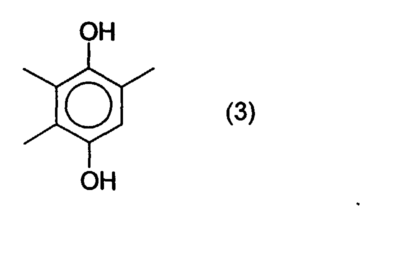 durch Umsetzung von 4-Oxo-isophoron (1) 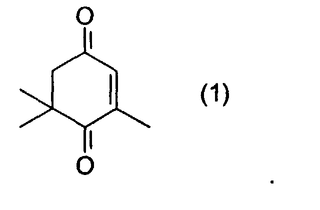 mit einem Acylierungsmittel in Gegenwart katalytischer Mengen einer Protonensäure und gegebenenfalls sich anschließende Verseifung des zunächst gebildeten Trimethylhydrochinonesters,
**dadurch gekennzeichnet,**
daß man als Protonensäure eine Säure mit einer Hammett-Konstante Hₒ < -11,9 (supersaure Säure) einsetzt.

2. Verfahren gemäß Anspruch 1,
**dadurch gekennzeichnet,**
daß man Perchlorsäure, Fluorsulfonsäure, Perfluoralkansulfonsäuren der allgemeinen Formel
CₙF₂ₙ₊₁SO₃H
in der n = 1-8 bedeutet einsetzt.

3. Verfahren gemäß Anspruch 1,
**dadurch gekennzeichnet,**
daß man Gemische von Lewissäuren und Brönstedtsäuren einsetzt.

4. Verfahren gemäß Anspruch 1,
**dadurch gekennzeichnet,**
daß man Mischungen von H₂SO₄ - H₃BO₃ oder HB(HSO₄)₄-H₂SO₄ einsetzt.

5. Verfahren gemäß Anspruch 1,
**dadurch gekennzeichnet,**
daß man Systeme einsetzt, die H⁺AlCl₄⁻ oder H⁺BF₄⁻ freisetzen.

6. Verfahren gemäß Anspruch 1,
**dadurch gekennzeichnet,**
daß man Magic Acid (HSO₃F-SbF₅) oder Fluor-Antimon-Säure (HF-SbF₅) einsetzt.

7. Verfahren gemäß einem oder mehreren der vorhergehenden Ansprüche,
dadurch gekennzeichnet, daß man die Säuren in einer Menge von 0,1 bis 50 Gew.-%, bezogen auf das Endion, einsetzt.

8. Verfahren gemäß einem oder mehreren der vorhergehenden Ansprüche,
dadurch gekennzeichnet, daß man pro Mol Keto-Isophoron >2 bis 4 Mol des Acylierungsmittels einsetzt.

9. Verfahren gemäß Anspruch 9,
dadurch gekennzeichnet, daß man Acetanhydrid, Diketen oder Acetylchlorid als Acylierungsmittel verwendet.

10. Verfahren gemäß einem oder mehreren der vorhergehenden Ansprüche,
dadurch gekennzeichnet, daß man das entstandene TMHQ-Diacetat ohne Isolierung, gegebenenfalls nach dem Abdestillieren von nicht umgesetztem Acetanhydrid, durch Zugabe von Wasser und/oder verdünnter Säure verseift und das entstandene TMHQ abtrennt.

11. Verfahren gemäß einem oder mehreren der vorhergehenden Ansprüche,
dadurch gekennzeichnet, daß man das entstandene TMHQ-Diacetat aus dem Reaktionsgemisch isoliert, und gegebenenfalls anschließend unter Verwendung verdünnter Säure in Gegenwart eines Phasenvermittlers verseift und das entstandene TMHQ abtrennt.

12. Verfahren gemäß Anspruch 12,
dadurch gekennzeichnet, daß man als Phasenvermittler Essigsäure, n-Butanol oder n-Butylacetat oder deren Gemische einsetzt.
